Europäisches Patentamt

European Patent Office

Office européen des brevets

(19)

(11) Publication number: **0 286 429**
**A1**

(12) # EUROPEAN PATENT APPLICATION

(21) Application number: 88303167.6

(22) Date of filing: 08.04.88

(51) Int. Cl.4: **C 12 N 15/00**
**A 01 H 1/00**

(30) Priority: 09.04.87 IL 82153

(43) Date of publication of application:
12.10.88 Bulletin 88/41

(84) Designated Contracting States:
AT BE CH DE FR GB IT LI LU NL SE

(71) Applicant: YISSUM RESEARCH DEVELOPMENT
COMPANY OF THE HEBREW UNIVERSITY OF
JERUSALEM
46 Jabotinsky Street
Jerusalem, 92 182 (IL)

(72) Inventor: Shilo, Judith
Levona Street 46
Gilo Jerusalem (IL)

Simchen, Giora
Rachel Imeniu Street 22
Jerusalem (IL)

Zamir, Daniel
Yavetz Street 2
Rehovot (IL)

(74) Representative: Marshall, Monica Anne et al
GALLAFENT & CO. 8 Staple Inn
London WC1V 7QH (GB)

(54) **Process for introducing genes into plants.**

(57) A process for introduction of an alien DNA into a target plant is described. Plasmid DNA containing a select alien DNA is applied to germinating pollen situated on mature stigmas of the target plant to effect the transformation of germinating pollen and incorporation of the plasmid DNA therein and into the genome of the resulting seeds, progeny plants, and fruits.

EP 0 286 429 A1

**Description**

## PROCESS FOR INTRODUCING GENES INTO PLANTS

The present invention relates to a process for introducing alien DNA into plants. More particularly the present invention relates to a process for introducing a chosen alien DNA into a target plant and into the progeny thereof.

Introduction of alien DNA into plants has been attempted in many laboratories to effect the genetic improvement of crops.

In the past the following systems have been proposed and/or developed:

(1) The use of Agrobacterium tumefaciens as a carrier.

As a soil bacterium, Agrobacterium has an afinity to wounded tissues of dicots. As reported by Chilton, M. D., Drummond M. H., Merlo, D. J., Sciaky, D., Montoya, A. L., Gordon, M. P., and Nester, E. W. (1977), Cell 11: 263-271, bacteria insert part of their genome, that naturally appears in a high molecular weight plasmid (Ti), into cells of the wounded tissue. Plasmid sequences are later found in other parts of the plant.

The Ti plasmid was studied extensively and genetically manipulated so that the penetration of the Ti plasmid into the plant genome does not cause any damage to the plant. Several genes were introduced by that system into plants and were found to be active when controlled by plant expression signals.

The main problem with this system is its specificity for dicot plants only; monocots are very rarely affected by Agrobacterium.

(2) The use of plant viruses as carriers.

Many viruses are known to cause plant diseases. It was found that the viruses insert their genetic material into the plant genome while causing the infection. The virus which was mostly studied is CaMV (cauliflower mosaic virus). Several genes were used to replace part of the viral genome in order to introduce them into the plant, for instance DHFR (dihydrofolate reductase).

(3) Plant protoplasts as recipients of alien DNA.

The discovery that plant cells can grow as protoplasts, without cell walls, led to several procedures for the introduction of alien DNA. Incubation of plant protoplasts with chemical fusagens such as PEG (polyethylene glycol) or PVA (polyvinyl alcohol) causes fusion of the membranes during which DNA from the outside may be caught in the protoplasts. For many crop plants, plant regeneration from protoplast tissue culture has not been achieved and, therefore, this method is not useful. Furthermore, somaclonal variants were found among regenerated plants as discussed hereinafter.

The main advantage of using plant protoplasts as alien DNA recipients is the ability to introduce very high molecular weight molecules and the non-specificity of the system.

(4) Lyposomes as carriers.

Artificial membranes made of lipids may be fused to protoplasts thus inserting molecules coated by the synthetic membrane into the plant protoplasts.

(5) Bacterial protoplasts as carriers.

Bacteria can form protoplasts under certain enzymatic conditions, thus by fusion with plant protoplasts they can transfer their own plasmids into the plants. In this way, Ti plasmid was transferred in the past from Agrobacter ium to plant protoplasts. (CaMV was also transferred into Saishin (Brassica chinensis L.) via E.coli protoplasts.

(6) Electroporation.

High voltage, when applied to cells, generates artificial pores in their membranes, through which high molecular weight compounds may be introduced. The system was used in mammalian cell culture (mice - as described by Kaspa, R. T., Teicher, L., Levine, B. J., Skoultch, A. I., Shafritz, D. A. (1986), Mol. Cell. Biol. 6: 716-718) and lately in plants.

(7) Microinjection.

Alien DNA may be introduced into mammalian cells by injection under the microscope. Mammalian cells are naturally attached to the surface of the growth chamber, thus allowing the needle to penetrate the cell and reach into the nucleus. Plant cells in culture are not attached to surfaces and are, therefore, not suitable for injection.

The two main disadvantages of the methods discussed above are as follows:

(a) Specificity - several methods are unique for one plant species (viruses) or for a family of plants (Agrobacterium).

(b) Somaclonal variation - all the methods which are based on plant tissue culture and regeneration result in new genetic variants, which are obtained even without the newly introduced DNA. The variants were shown to contain chromosomal changes (deletions, translocations, etc.), for example in potatoes as reported by Greissen, G. P., Karp, A. (1985), Plant Cell Tissue &; Organ Culture 4:171-182; in wheat as reported by Karp, A., Bright, S. W. J. (1985), Molec. Cell. Biol. 2: 199-234; in tobacco as reported by Gengenbach, B. G. (1985), in Int. Symp. Genetic Manipulation In Crops, Beijing, China, ed. Li, P.

Similarly, point mutations were found in tomatoes as reported by Evans, D. A., Sharp, W. R. (1983), Science 221: 949-951; and in lemon, rice, and onion, etc., as reported by Larkin, P. J., Scowcroft, W. R. (1981), Theor. Appln. Genet., 60: 194-214.

One or more of the following reasons are usually given for somaclonal variation:

(a) The variation exists naturally in the plant tissue before the transfer into culture, and is only revealed by the in-vitro conditions.

(b) The variation is caused by chemicals present in the tissue culture media.

(c) The variation is due to "genetic shock" which results from the transfer to tissue culture conditions, and/or regeneration from tissue into mature plant.

The first explanation is strengthened by the finding of chromosome variation along the stem of the plant.

The second explanation is supported by the finding that the longer the plant cells are in culture conditions, the more variation is found. Furthermore, various compounds that are present in the culture medium are mutagenic, for instance the hormones 2,4D or NAA.

The third explanation is based on the findings of transposable elements in the genome of plants.

McClintock, B., (1950) Proc. Nat. Acd. Sci., 36: 344-355, found that these elements in corn "jump" from one place to another in the genome following a "genetic shock." In her case the "shock" was caused by crossing relatively unrelated varieties of corn. It has been proposed that culturing somatic cells and/or regeneration similarly constitute a "genetic shock" which may induce transpositions and, thus, generate the somaclonal variation.

In light of the state of the art and the failure thereof to overcome the disadvantages mentioned above, it is now proposed according to the present invention to ameliorate these problems by providing a process for introducing a chosen alien DNA into a target plant comprising applying plasmid DNA, containing said chosen alien DNA, to germinating pollen situated on mature stigmas of said plant to effect the transformation of said germinating pollen and the incorporation of the plasmid DNA therein and into the genome of resulting seeds, progeny plants and fruits.

In preferred embodiments of the present invention, said plasmid DNA incorporates a DNA sequence of the targeted plant species which mediates the integration of the plasmid DNA into the genome of the targeted plant.

Thus, the present invention is also directed to seeds, plants, and fruits having alien DNA integrated in the genome thereof whenever produced by the process of the present invention.

The use of germinating pollen grains as the recipients and transfer vehicle of alien DNA has several advantages including the following:

(a) Pollen grains are very well characterized biochemically.

(b) The tip of the pollen tube lacks callose - the polysaccharide molecule which serves as the barrier to foreign molecules and prevents the latter from penetrating plant cells.

(c) Very extensive biochemical activity was found in the germinating pollen: protein synthesis, RNA synthesis, and DNA repair-like synthesis.

(d) Selection was found to take place among germinating pollen grains of different genotypes. In corn, when pollen from a waxy mutant were mixed with wild-type pollen before fertilization, the mutant pollen were selected against and only a low portion of progeny contained the wx allele. Another example in which certain selectable genes were presumably expressed during pollen germination concerns the adaptability to low temperatures: Pollen of tomatoes from the heights of Peru (L.hirsultum) had a three-fold selective advantage over pollen of a common variety (L.esculantum) when pollination was done in low-temperature conditions.

In order to illustrate the new methodology of the present invention, work was carried out with germinating pollen tubes of tomato. The tomato system is well characterized and has the following advantages:

(a) Natural diversity is found within and between species.

(b) The plant is easy to grow in various weather conditions.

(c) There is a detailed genetic map of the tomato including alleles of various protein isozymes.

(d) Mutations of various kinds are known - natural and induced.

(e) Male sterile strains are available.

(f) The flower is large enough for easy manipulations.

(g) Cross- or self-pollination may be applied, as well as asexual propagation.

(h) A short generation time.

(i) Large number of seeds may be found in each fruit.

(j) The 12 chromosomes may be clearly identified cytologically during the pachytene stage of meiosis.

(k) Molecular biology methods have already been used in tomato and other species of the Solanacea family.

Introduction of alien DNA into germinating pollen was reported by Hess, D. (1979), Z. Pflanzen Physiol. 93: 429-436; and Hess. D. (1980), Z. Pflanzen Physiol. 98: 321-337. However, the papers by Hess describe pollen transformation that was done in vitro: the pollen grains were germinated in vitro, incubated with alien DNA and, only later, used to pollinate stigmas of the recipient plant. Hess did not prove that the DNA he introduced to the plant was present in the offsprings and no reports of further experiments along these lines have been reported. Furthermore, as shown in Comparative Example A hereinafter, in fact attempts to effect pollen transformation in vitro is much less effective than the process of the present invention.

A year ago there was published a paper of Ohta, Y. (1986), Proc. Natl. Acad. Sci. U.S.A. 83: 715-719, describing high efficiency genetic transformation of maize by a mixture of pollen and exogenous DNA. In his experiments, Ohta mixed DNA of a donor strain with pollen of a recipient maize, and then used the pre-prepared mixture for self-pollination. Approximately 9% of the kernels had some transformed endosperm, based on the appearance of color markers from the donor plant. However, it is clear that only very few embryos were transformed. Furthermore, Ohta did not prove physical presence of the donor DNA.

The present system is different from Ohta's in two major ways. Firstly, as described hereinafter, the presence of the inserted DNA has been proved, by hybridization to a probe which is specific for the alien plasmid that was introduced.

Secondly, in the present process, the stigmas are first pollinated with dry pollen and only afterwards is the alien DNA introduced to the pollinated stigmas, as opposed to Ohta, whose liquid mixture might result in initial undesirable germination in vitro.

Thus, in fact none of said references teach or suggest the process of the present invention.

Furthermore, as can be seen in the examples hereinafter, the present system for introducing DNA is very efficient - 11% of the fruits obtained from such pollinations contained 1-6 seeds each, which gave rise to plantlets carrying the alien DNA.

The system is based on transformation during pollination, thus the plant is never exposed to in vitro conditions and somaclonal variation is avoided.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

## Examples

### (A) General Methods, Procedures, and Discussion:

In all experiments, the DNA that was applied to germinating pollen tubes was plasmid DNA. The main reason for choosing this type of DNA molecule was that it is easy to obtain in the form of a homogeneous preparation.

The plasmids were constructed by combining the Kanamycine Phosphotransferase gene with the yeast ARSI/TRPI sequence. The new plasmid pJS-6 was built by inserting a 1.7 Kb sequence originating from plasmid CV13:Tn903 (Grindley, N. D., Joyce C. M., Proc. Natl. Acad. Sci. U.S.A. 77: 7176-7180) which codes for Kanamycine resistance, into the PvuII site of the yeast autonomously replicating plasmid YRP7 (Tschumper, G., Carbon J. C. (1980), Gene 10: 157-166).

The 1.7 Kb fragment was cut out of CV13:Tn903 using the PvuII restriction enzyme. The new plasmid was used to transform E.coli strain JA194 (an HB101 strain containing the mutation trpC- which can be complemented by the yeast gene TRPI) under selective conditions - no tryptophane in the M9 medium to which the antibiotic Ampicilin was added (50 µg/ml). Propagation of the plasmid was done according to Maniatis, T., Fritsch, G. F., Sambrook, J. (1982), Cold Spring Harbor, New York.

Various strains of tomatoes were tested for their ability to germinate in-vitro. $10^8$ grains were suspended in 10 ml of SB (18% sucrose + 0.015% boric acid). After two hours of slow rotation (100 rpm) at room temperature, the pollen grains were examined under the microscope for pollen tube growth. L.hirsutum strain LA 1777 was chosen for further experiments because with the conditions described above it gave the highest percentage of in-vitro pollen germination.

### (B) Introduction of Plasmid DNA Into Germinating Pollen

Freshly collected pollen of L.hirsutum were suspended in SB liquid medium under the conditions described above. When about 50% of the pollen grains showed the beginnings of tube development (time 0), the suspension was divided into three and plasmid DNA was added to two of the three suspensions. After a further incubation for 1 hour, each sample was divided into two, and to one-half 0.1 mg/ml of antibiotic G-418 was added. Each hour (starting at time 0), a 50 µl sample of each treatment was examined under the microscope and further elongating pollen tubes were counted. 400 grains were examined in each sample. The pollen were characterized as follows: (1) further elongating tubes; (2) non-elongating and/or dead cells.

The experiment was repeated several times and the results (percentage of further elongating tubes out of 400 grains) of one experiment are given in Table 1.

## T A B L E   1

### Percentage Of Further Elongating
### Pollen Tubes Treated With Plasmid DNA

| Time | No. DNA | | + YRP7 | | + pJS-6 | |
|------|---------|--|--------|--|---------|--|
| 0 | 50 | | 50 | | 50 | |
| 1 | 55 | | 47 | | 50 | |
| | G-418 | Control | G-418 | Control | G-418 | Control |
| 2 | 43 | 48 | 42 | 48 | 51 | 47 |
| 3 | 35 | 57 | 37 | 45 | 44 | 44 |
| 4 | 28 | 60 | 28 | 47 | 45 | 43 |

The inhibitor G-418 was added to one-half of each preparation one hour after the addition of DNA. The other half served as control.

The results given here show that the presence of the plasmid pJS-6 allows further elongation of the pollen tube when exposed to G-418, whereas the presence of YRP7 does not allow it.

Furthermore, protein extracts isolated from treated germinating pollen were tested for the activity of the enzyme coded by the plasmid - Kanamycine Phosphotransferase. Proteins were prepared from germinating pollen using the following procedure:

(1) Treated pollen grains were washed twice with SB, resuspended in 1 mM EDTA + 14 mM mercaptoethanol (1:20 of the original volume) and sonicated $5 \times 20$ seconds on ice.

(2) The broken cells were then pelleted in an Eppendorf centrifuge at a high speed for 15 minutes.

(3) Kanamycine Phosphotransferase activity (cpm) was measured according to Haas, M. J., Dowding, J. E. (1975), Meth. Enzym. 43: 611-628, and the concentration of protein was determined according to Lowry, O. H., Rosebrough, N. J., Farr, A. L., Randall, R. J. (1951), J. Biol. Chem. 193: 265-275.

Several experiments were done comparing the enzymatic activity in proteins extracted from treated and non-treated pollen as set forth in the following Table 2.

## T A B L E   2

### Kanamycine Phosphotransferase Activity
### In Protein Extracts

| | Treatment | Activity cpm | g Protein | Specific Activity cpm/µg Protein |
|--|-----------|--------------|-----------|----------------------------------|
| 1. | no DNA | 639 | 85 | 7.5 |
| 2. | YRP7 | 440 | 55 | 8.0 |
| 3. | YRP7 + G-418 | 822 | 80 | 10.3 |
| 4. | pJS-6 | 505 | 15 | 33.6 |
| 5. | pJS-6 + G-418 | 1509 | 35 | 43.1 |

Basal activity was found in proteins isolated from untransformed pollen and in proteins isolated from pollen transformed with YRP7. Four times more activity was found in proteins extracted from pollen into which the plasmid pJS-6 was introduced. When pollen was exposed to G-418 two hours prior to protein extraction, Kanamycine Phosphotransferase activity was 10% higher than the activity found in proteins that were extracted from treated pollen that were not exposed to G-418. This activity can be explained as non-specific Phosphotransferase activity.

### (C) The Fate Of The Incorporated DNA

For locating the DNA that was introduced into the germinating pollen, a radioactive probe (pBR322) was hybridized to pollen DNA. DNA was isolated from treated germinating pollen using the following procedure:

1. Treated pollen grains were washed twice with SB.

2. The pellet was resuspended in 400 μl buffer containing 50 mM tris-HCl; pH 8.0; 1 mM EDTA, 14 mM -mercaptoethanol at 0°C.

3. Sarkosil at a final concentration of 1% was added and the mixture was incubated at 65°C for 30 minutes.

4. 80 μl of 5M KAc was added for an incubation at 0°C for 1 hour.

5. The mixture was spun down in an Eppendorf centrifuge for 15 minutes for removal of non-germinated pollen grains and cell walls.

6. The supernatant was transferred to a new Eppendorf tube and equal volume of isopropanol was added and mixed well.

7. After 10 minutes of incubation at room temperature, the DNA was pelleted in an Eppendorf centrifuge for 15 minutes.

8. The DNA was resuspended with 10 μl $H_2O$; 25 μl 10M $NH_4Ac$ was added and mixed, and 2 volumes of isopropanol was further added and mixed well.

9. After 10 minutes at room temperature, the DNA was further centrifuged and washed with 70% ethanol at 4°C.

10. The DNA was dried and then resuspended in 50 μl TE (10 mM tris-HCl; pH 8.0, 1 mM EDTA).

The DNA was digested with restriction enzymes and separated by electrophoresis in an agarose gel, transferred to a nitrocellulose filter (as described by Southern, E. M. (1975), J. Mol. Biol. 98: 503-517), and hybridized to a pre-labeled plasmid DNA (pBR322), a sequence that is present on the inserted plasmid but not in the tomato DNA.

Hybridization patterns showed that the plasmid that entered through the tip of the pollen tube found its way to the nucleus and integrated into the plant genome. The frequency of the integration was very low - calculated from the requirement for very high specific activity of the probe (had to be at least $5 \times 10^8$ cpm), and from the length of the exposure time of the hybridized filter to the X-ray film (7 days or more).

For overcoming the problem of the low frequency of integration, a new plasmid pJS-100 was constructed based on pJS-6. It was previously found that for enhancing integration of plasmid DNA in yeast, a piece of the yeast DNA was included in the transforming plasmid; this enabled the whole plasmid to integrate into the yeast genome by homologous recombination (Orr Wever, T. L., Szostak, J. W., Rothstein, R. J. (1981), Proc. Natl. Acad. Sci. U.S.A. 78: 6354-6358). Along similar lines of reasoning, chromosomal DNA from tomato (L.esculantum) was prepared (as described by Frankel, R., Scowcroft, W. R., Whitfeld, P. R. (1979), Mol. Gen. Genet. 169: 128-135); and a piece of 12.5 Kb from this DNA was cloned into the BamHI site of pJS-6, as described by Maniatis, ibid.

Indeed, the presence of the host DNA in the plasmid enhanced the integration frequency so that $5 \times 10^7$ cpm and 24 hours of exposure were sufficient for showing clear presence of the plasmid into the plant genome. The integration was found to be non-specific even though the tomato DNA sequence that was cloned into the plasmid showed unique hybridization pattern to tomato nuclear DNA, and is therefore presumed to originate from unique, rather than from repetitive, sequences.

### Comparative Example A

In order to define the best conditions for plant transformation, a semi-in-vivo experimental system was studied. The questions that were asked were, 1st - are pollen germinated prior to pollination able to pollinate as efficiently as dry pollen? 2nd - does DNA interfere with pollination? and 3rd - will a selection during pollination be efficient?

For answering these questions, isolated fresh flowers were emasculated (the filaments carrying the anthers were removed) and were pollinated as follows:

a) Pollination with dry pollen - control.

b) Pollination with dry pollen with the addition of 0.5 μl plasmid DNA (in solution, 2.5 μg/μl).

c) Pollination with pre-germinated pollen (germinated in-vitro).

d) Pollination with pre-germinated pollen that were transformed in-vitro with plasmid DNA.

e) Pollination with dry pollen through a 0.5 mm layer of agarose.

f) Pollination with dry pollen through a 0.5 mm layer of agarose containing 0.1 mg/ml G-418.

g) Pollination as in f) with the addition of 0.5 μl plasmid DNA.

Following the treatment, the flowers were put in a 25°C growth chamber (95% humidity) for 24 hours. The pistils were then cleaned off from the extra materials and put in 8M NaOH for 24 hours; after which they were washed thoroughly in water and put on a microscope slide in 0.2% Anillin Blue dissolved in 0.1M $K_3PO_4$.

After 5 minutes, the pistils were covered with cover lass and squashed gently between 3MM papers.

Under UV light, the pollen tubes were observed - on the stigma, through the pistils and within the ovaries. Pollination e), f), and g) were unsuccessful - no pollen tube could be observed penetrating the agarose, indicating that a selection during pollination is not effective. In pollination c) and d), only very few pollen tubes were observed through the pistils and only 3-5 were seen within the ovaries. These results led to the conclusion that in-vitro germination prior to pollination should be avoided as the pre-germinated pollen do not maintain their in-vivo pollination capability.

In pollination a) and b), many pollen tubes were observed along the pistils and more than 50 of the tubes were found within the ovaries. No difference was observed between pollinations a) and b), or between c) and d) - the presence of plasmid DNA at the concentration that was tested did not have any effect on pollination efficiency.

Example 1

12 pots with flowering plants of L.esculantum strain Koren which carry a nuclear male sterility mutation were transferred to a growth chamber (25°C, 95% humidity) 24 hours prior to the pollination. Fresh pollen was collected from L.esculantum M-88 and was used for pollinating 300 emasculated flowers (no anthers and petals or septals). On top of the pollinated stigmas, 2 µl of 2.5 µg/µl plasmid (pJS-100) DNA were placed, using a Gilson micropipet. The treated tomato plants were then put back to the growth chamber (same conditions as before) for 24 hours for the pollination to take place at the best conditions. The plants were then transferred to the greenhouse for fruit development.

One-hundred and seventy-five (175) fruits were collected 5-7 weeks after pollination and seeds were isolated from each (1-60 seeds/fruit). The isolated seeds were incubated with the juice of the fruit for 3-5 days for fermentation, after which the seeds were washed with water and dried in the air. Seeds may be kept under these conditions for a long time.

3832 seeds (representing 99 fruits) were sown under greenhouse conditions 20-25°C, 80-90% humidity. Three to five days after sowing, plantlets could be observed; and, 20 days later, DNA was prepared from the young leaves for screening for the sequence that was previously introduced during pollination.

The plants were divided into groups of about 10 for preparation of DNA as follows:

1. 0.1 gr of freshly collected leaves (representing around 10 plants) were put in a plastic bag and sealed.

2. The sealed bags were frozen in liquid nitrogen and squashed.

3. The squashed pieces of leaves were then transferred into ceramic mortars with liquid nitrogen and further crushed into powder.

4. When the remainder of the nitrogen evaporated, the powder was transferred into Eppendorf tubes with 1 ml buffer A (0.5M NaCl, 50 mM EDTA, 100 mM tris-HCl; pH 8.0, 10 mM -mercaptoethanol) in each tube.

5. The mixture was sonicated 3-5 times for 15 seconds each (on ice) until a homogenized solution was obtained.

6. 0.1 ml of 20% SDS (sodium dodecil sulfate) was added and mixed well.

7. After 10 minutes incubation at 65°C, 0.1 ml of 5M KAc was added.

8. After 20-30 minutes on ice, the mixture was centrifuged at high speed in the Eppendorf centrifuge for 30 minutes.

9. The supernatant was transferred to a new Eppendorf tube through a Miracloth paper filter and an equal volume of isopropanol was added and mixed well.

10. After a 20 minute incubation at -20°C, the mixture was centrifuged for 20 minutes.

11. The soft pellet was resuspended in 70 µl buffer B (10 mM EDTA, 50 mM tris-HCl, pH 8.0) and centrifuged for 10 minutes for removing the non-solubles.

12. The supernatant was transferred to a new Eppendorf tube into which 7.5 µl of 3M NaAC; pH 5.8, was added with the addition of 0.1 ml isopropanol.

13. 30 seconds centrifugation pelleted the DNA which was washed with cold 70% ethanol and dried.

14. The dry DNA was dissolved in 50 µl TE (10 mM tris-HCl; pH 8.0, 1 mM EDTA).

15. 10 µl of the DNA solution was used for further experiments.

The DNA was used for loading a dot-blot as described by Saxena, P. K., Mii, M., Crossby, W. L., Fowke, L. C., and King, J. (1986), Planta 168: 29-35, and was then hybridized with a probe of labeled pBR322 according to Maniatis, ibid.

Out of 320 DNA preparations that were done (representing the 2303 plants), 27 showed presence of pBR322 sequences. DNA was prepared from 273 plants for further identification, and 48 plants showed positive reaction to a pBR322 probe. Data describing the distribution of the presence of pBR322 sequences among the tomato fruits is presented in Table 3.

## T A B L E   3

### The Distribution Of The pBR322 Sequence In The Different Plants

| Tomato No. | No. of Plants That Were Tested | No. of Plants That Showed Presence Of pBR322 Sequences |
|---|---|---|
| 20 | 51 | 7 |
| 45 | 22 | 5 |
| 46 | 7 | 3 |
| 73 | 10 | 1 |
| 86 | 33 | 10 |
| 88 | 24 | 1 |
| 94 | 1 | 1 |
| 95 | 47 | 4 |
| 97 | 12 | 7 |
| 98 | 33 | 8 |
| 99 | 11 | 1 |

The results in Table 3 demonstrate the high efficiency of the transformation under the conditions constructed. 11% transformation frequency if each pollination reflects a transformation system and 0.25% if one refers to each pollen grain as to a transformation system. According to the distribution frequency - if in one plant of a certain fruit the pBR322 presence was present, the probability to find it among plants originating from the same fruit was higher than the probability to find it in others.

Further identification of the nature of the insertions of the alien DNA into the plant genome were done in two ways:

(1) DNA was further prepared from several plants according to Frankel, ibid.

The DNA was cut by restriction endonucleases, separated on an agarose gel electrophoresis and trans ferred to a nitrocellulose filter that was further used for hybridization with $^{32}$P-nick-translated pBR322.

(2) The plasmid pJS-100 was separated to five fragments as described in Table 4. The isolated fragments were used to determine whether all the fragments are found within the plant DNA or some of them are lost.

**0 286 429**

## T A B L E   4

### Separation Of pJS-100 To Fragments

| Fragment No. | Size | Includes | Cut By | Origin* |
|---|---|---|---|---|
| 1 | 752 pB | Amp | EcoRI/PstI | pBR322 |
| 2 | 1.4 Kb | ARSI/TRPI | EcoRI | YRP7 |
| 3 | 375 bp | pBR322 | EcoRI/BamHI | pBR322 |
| 4 | 1.5 KB | Ori | PstI/PvuII | pBR322 |
| 5 | 1.7 KB | Kanamycine | PvuII | CV13:Tn903 |

* The fragments were isolated from the original plasmids so that no contaminations of undesired sequences (such as tomato DNA) will effect the results.

The fragments were obtained from the plasmids mentioned above as follows:
1. Plasmid DNA is cut with the desired restriction enzyme.
2. The DNA is separated in an agarose gel electrophoresis.
3. The fragment DNA is cut out of the gel and incubated in buffer A (10 mM tris-HCl; pH 8.0, 1 mM EDTA, 5 mM NaCl) for 30 minutes. The DNA is then electroeluted into a trap containing 0.25% Bromo Phenol Blue, 0.25% xylene cyanol, and 15% Ficol (type 400) dissolved in 3M NaAc; pH 5.6.
4. 400 µl of solution from the trap is removed into an Eppendorf tube and the DNA is precipitated as described in Maniatis, ibid.
5. The isolated fragment DNA is then dissolved in the desired buffer for further use.

The fragments were used as probes. Each fragment was separately labeled radioactively (Maniatis), and hybridized to a nitrocellulose filter loaded with dots of DNA as follows:
1) Treated Tomato No. 45-2
2) Treated Tomato No. 45-5
3) Treated Tomato No. 45-11
4) Untreated L.esculantum
5) No DNA
6) pJS-100

Five such filters were prepared and each one of them was hybridized with a different fragment.

Each of the five blots was hybridized with a different probe; the five probes together cover most of the vector. The results prove that all of the sequences that were tested for are present in the plants, namely, most of the vector is found in the genome of the treated plants. As expected, the sequences were not found in the untreated tomato DNA.

From the experiments described above, it can be concluded that the plasmids DNA is integrated in the genome of the plants in one copy or more. Furthermore, none of the fragments that were checked was lost while integrating.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

**Claims**

1. A process for introducing a chosen alien DNA into a target plant comprising applying plasmid DNA, containing said chosen alien DNA, to germinating pollen situated on mature stigmas of said plant to effect the transformation of said germinating pollen and the incorporation of the plasmid DNA therein and into the genome of resulting seeds, progeny plants and fruits.

2. A process according to claim 1 wherein said plasmid DNA incorporates a DNA sequence of the targeted plant species which mediates the integration of the plasmid DNA into the genome of the targeted plant.

3. Plants having alien DNA integrated in the genome thereof whenever produced by the process of claim 1.

4. Fruits having alien DNA integrated in the genome thereof whenever produced by the process of claim 1.

5. Seeds having alien DNA integrated in the genome thereof whenever produced by the process of claim 1.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,Y | PROC. NATL. ACAD. SCI., vol. 83, February 1986, pages 715-719; Y. OHTA: "High-efficiency genetic transformation of maize by a mixture of pollen and exogenous DNA" * abstract; table 1; page 719 * | 1,3-5 | C 12 N 15/00<br>A 01 H 1/00 |
| Y | WO-A-8 501 856 (DE WET) * page 8, line 35 - page 9, line 18; page 15, lines 2-4,15-26; claims 1-5 * | 1,3-5 | |
| A | EP-A-0 204 590 (INSTITUT NATIONAL DE LA RECHERCHE AGRONOMIQUE) * page 2, lines 5-10; claim 14 * | 2 | |
| A . | CHEMICAL ABSTRACTS, vol. 94, no. 13, 30th March 1981, page 451, abstract no. 99947q, Columbus, Ohio, US; D. HESS: "Attempts to transfer kanamycin resistance of bacterial plasmid origin in Petunia hybrida using pollen as vectors", & BIOCHEM. PHYSIOL. PFLANZ. 1981, 176(4), 324-330 * abstract * | 1,3-5 | TECHNICAL FIELDS SEARCHED (Int. Cl.4)<br><br>C 12 N 15/00<br>A 01 H 1/00 |

The present search report has been drawn up for all claims

| Place of search | Date of completion of the search | Examiner |
|---|---|---|
| BERLIN | 29-06-1988 | JULIA P. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
..........................................................................................
& : member of the same patent family, corresponding
document

EPO FORM 1503 03.82 (P0401)